(19) Europäisches Patentamt / European Patent Office / Office européen des brevets

(11) **EP 2 962 729 A1**

(12) **EUROPEAN PATENT APPLICATION**
published in accordance with Art. 153(4) EPC

(43) Date of publication:
**06.01.2016 Bulletin 2016/01**

(51) Int Cl.:
*A61N 7/00* (2006.01)    *A61B 8/00* (2006.01)
*A61B 8/14* (2006.01)    *G01S 15/89* (2006.01)

(21) Application number: **13876470.9**

(22) Date of filing: **28.02.2013**

(86) International application number:
**PCT/KR2013/001671**

(87) International publication number:
**WO 2014/133210 (04.09.2014 Gazette 2014/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Alpinion Medical Systems Co., Ltd. Hwaseong-si, Gyeonggi-do 445-380 (KR)**

(72) Inventors:
• **SON, Keonho**
  **Seongnam-si**
  **Gyeonggi-do 463-440 (KR)**

• **KANG, Kookjin**
  **Yongin-si**
  **Gyeonggi-do 448-536 (KR)**
• **KIM, Daeseung**
  **Seoul 157-882 (KR)**
• **KIM, Myungdeok**
  **Seoul 152-050 (KR)**
• **JUN, Sukhwan**
  **Incheon 402-200 (KR)**

(74) Representative: **Thorniley, Peter et al Kilburn & Strode LLP 20 Red Lion Street London WC1R 4PJ (GB)**

(54) **METHOD FOR CONFIRMING LOCATION OF FOCAL POINT, AND ULTRASONIC MEDICAL APPARATUS THEREFOR**

(57)     A method and an ultrasound medical apparatus for confirming a focal point of a high-intensity focused ultrasound are disclosed. A method of confirming a focal point, for pre-targeting a location of a high-intensity ultrasound by synchronizing the high-intensity ultrasound and an imaging ultrasound and using a reflected signal of the synchronized imaging ultrasound and high-intensity ultrasound from a focal point to which the ultrasounds are transmitted, and an ultrasound medical apparatus for implementing the method are provided.

**FIG. 1**

**Description**

[Technical Field]

The present disclosure relates to a method for confirming a focal point and an ultrasound medical apparatus therefor, and more particularly, to a method for confirming a focal point, for the purpose of pre-targeting the location of a high-intensity ultrasound by synchronizing the generation time of the high-intensity ultrasound with that of an imaging ultrasound and using reflected signals of the synchronized imaging and high-intensity ultrasounds from a focal point to which the ultrasounds are transmitted, and an ultrasound medical apparatus for implementing the method.

[Background]

The statements in this section merely provide background information related to the present disclosure and do not necessarily constitute prior art.

An ultrasound is a sound wave having a frequency higher than an audio frequency. The ultrasound has a high energy, and as a wave, it can be focused on a point. A human tissue is necrotized at a temperature of 60°C to 85°C, and hence, when a thermal or mechanical energy is applied to a tissue by increasing the intensity of the ultrasound and focusing it on a point, the corresponding tissue can be removed. This is referred to as a high-intensity focused ultrasound (HIFU) (hereinafter, a "high-intensity ultrasound") treatment.

Using such a high-intensity focused ultrasound eliminates a medical procedure such as an abdominal operation. Compared to a surgical operation and a chemical treatment, a non-invasive treatment can be achieved, which causes less damage to a patient.

On the other hand, with the treatment using the high-intensity ultrasound based on the premise to destroy or degenerate a treatment site of a patient, imprecisely controlled focusing can destroy or degenerate areas other than the treatment site. Therefore, the precise focal point of the high-intensity ultrasound needs to be confirmed before or while performing a treatment using the high-intensity ultrasound.

[Disclosure]

[Technical Problem]

The present disclosure has been made in an effort to effectively resolving at least a part of the above-mentioned problems, and it is an object of some embodiments of the present disclosure to provide a method of confirming a focal point, for the purpose of pre-targeting the location of a high-intensity ultrasound by synchronizing the generation time of the high-intensity ultrasound with that of an imaging ultrasound and using reflected signals of the synchronized imaging and high-intensity ultrasounds from a focal point to which the ultrasounds are transmitted, and an ultrasound medical apparatus for implementing the method.

[Summary]

According to some embodiments of the present disclosure, an ultrasound medical apparatus is provided, which includes an imaging transducer including a plurality of transducer elements each configured to transmit an imaging ultrasound to a target object, a plurality of treatment transducers each configured to transmit a high-intensity ultrasound to a focal point corresponding to a specific focal-point information of the target object, a synchronizing unit configured to synchronize transmission times of the imaging ultrasound and the high-intensity ultrasound to render the imaging ultrasound and the high-intensity ultrasound to reach the focal point at the same time, and an image processing unit configured, after the imaging ultrasound and the high-intensity ultrasound are transmitted in a synchronized manner, to generate an image picture based on echo signals received by the imaging transducer.

According to another embodiment of the present disclosure, a method is provided for confirming a focal point on an image picture in an ultrasound medical apparatus including an imaging transducer including a plurality of transducer elements each configured to transmit an imaging ultrasound and a plurality of treatment transducers each configured to transmit a high-intensity ultrasound, the method including synchronizing transmission times of the imaging transducer and the treatment transducers to render the imaging ultrasound and the high-intensity ultrasound to reach the focal point at the same time, and generating, after the imaging ultrasound and the high-intensity ultrasound are transmitted in a synchronized manner, the image picture based on an echo signal received by the imaging transducer.

[Advantageous Effects]

According to some embodiments of the present disclosure as described above, a focal point, for pre-targeting a location of a high-intensity ultrasound, can be confirmed by synchronizing the generation times of the high-intensity and imaging ultrasounds and using reflected signals of the synchronized imaging and high-intensity ultrasounds from a focal point to which the ultrasounds are transmitted.

Further, according to some embodiments, by synchronizing generation times of the high-intensity ultrasound and the imaging ultrasound, an ultrasound image displaying the focal point of the high-intensity ultrasound can be acquired, and thereby possibly improper focal point can be automatically compensated.

Moreover, according to some embodiments, the correct and precise focal point can be confirmed in the course of a treatment, as well as before performing the treatment.

[Brief Description of Drawings]

FIG. 1 is a schematic block diagram of an ultrasound medical apparatus according to some embodiments of the present disclosure.

FIG. 2 is a schematic block diagram of an image processing unit according to some embodiments.

FIGs. 3A and 3B are schematic diagrams for illustrating a beam forming process by a transducer array according to some embodiments.

FIG. 4 is a schematic diagram for illustrating a reception directivity of the transducer array according to some embodiments.

FIGs. 5A and 5B are schematic diagrams for illustrating a process of generating an ultrasound by a transducer unit under the control of a synchronizing unit according to some embodiments.

FIGs. 6A-6C are schematic diagrams of a virtual channel and a treatment channel according to some embodiments.

FIG. 7 is a schematic diagram for illustrating an actual application of the reception directivity according to some embodiments.

FIG. 8 is a schematic diagram of an image picture displaying a focus and a graph of a reception signal in a focusing direction received by an imaging transducer according to some embodiments.

FIG. 9 is a flowchart of a method for confirming a focal point of a high-intensity ultrasound according to some embodiments.

REFERENCE NUMERALS

| | |
|---|---|
| 110: Treatment Transducer | 120: Imaging Transducer |
| 125: Transducer Unit | 130: Image Processing Unit |
| 140: Synchronizing Unit | 150: Control Unit |
| 160: Storage Unit | 170: Input Unit |
| 180: Display Unit | 210: First Transmit Beamformer |
| 220: Second Transmit Beamformer | 230: Transmit Beamformer |
| 240: Receive Beamformer | 250: Beamformer Unit |
| 260: Signal Processing Unit | 270: Scan Converter |
| 310: Delay Circuit | 320: Summing Circuit |
| 510: Focal Point | 610: Virtual Channel |
| 620: Treatment Channel | |

[Detailed Description]

Hereinafter, at least one embodiment of the present disclosure will be described in detail with reference to the accompanying drawings.

In some embodiments, in order to confirm (pre-target) a precise focal point of a high-intensity ultrasound, the high-intensity ultrasound is transmitted in a form of pulse, and a reflected wave in the form of pulse is received and imaged by an imaging transducer 120. A scanline described in some embodiments refers to a region where the imaging transducer 120 transmits and receives an imaging ultrasound, which is equivalent to a graphic area generated by a single transmission and reception.

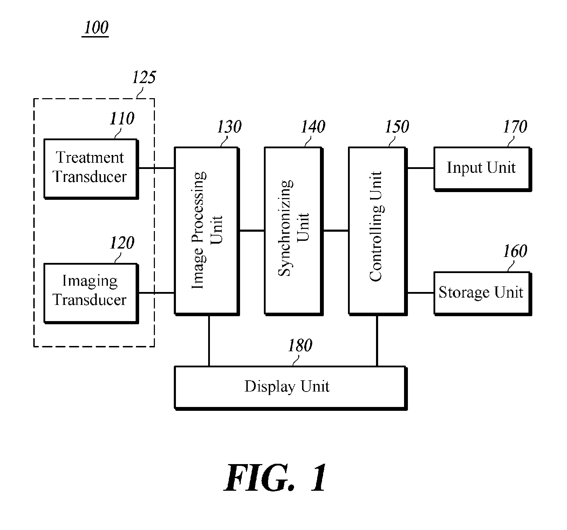

FIG. 1 is a schematic block diagram of an ultrasound medical apparatus according to some embodiments.

An ultrasound medical apparatus 100 includes a transducer unit 125, an image processing unit 130, a synchronizing unit 140, a control unit 150, a storage unit 160, an input unit 170 and a display unit 180.

The transducer unit 125 converts an input electrical signal into an ultrasound or vice versa. The transducer unit 125 includes a treatment transducer 110 and an imaging transducer 120 attached or provided on a support unit (not shown).

The treatment transducer 110 generates a high-intensity ultrasound in a periodic manner based on a control signal inputted from the image processing unit 130, and transmits the high-intensity ultrasound to an affected area (focal point). According to some embodiments, the treatment transducer 110 generates the high-intensity ultrasound in a form of pulse with an intensity within a range that does not destroy a tissue of a treatment region, in order to confirm a precise focal point 510. However, when the control unit 150 confirms the focal point as a correct location, the treatment transducer 110 transmits a continuous high-intensity ultrasound for treatment to the focal point. The treatment transducer 110 is controlled to focus the high-intensity ultrasound on the focal point 510. The treatment transducer 110 is not necessarily a transducer array since it needs no such transducer elements for rapidly and precisely controlling the direction of the ultrasound, although such a transducer array may be used as the treatment transducer 110 in order to precisely control or easily change the focal point.

The transducer array refers to a transducer including a plurality of transducers in an array and generating or receiving an ultrasound in response to an electrical signal. Therefore, each transducer that generates the ultrasound in the transducer array is a separate transducer; however, each transducer that generates the ultrasound will be referred to as a transducer element in order to distinguish it from the transducer array. The transducer array is configured to control ultrasound generation timing of each transducer element so as to change a direction of the ultrasound, when an image may be generated by grouping the transducer elements dedicated to different scanlines from each other.

The imaging transducer 120 generates an imaging ultrasound based on the control signal inputted from the image processing unit 130, and transmits the imaging ultrasound in a designated direction. Further, the imaging transducer 120 receives and converts a wave reflected from the designated direction into an electrical signal, and transmits the same to the image processing unit 130. The imaging transducer 120 transmits the imaging ultrasound in a straight line and receives the reflected wave in order to generate a 2D image of a region to be ultrasound imaged. Hence the imaging transducer 120 receives a single straight line image per transmission of the imaging ultrasound. The imaged region from a single cycle of the imaging ultrasound transmission and reception is called a scanline. An image of a single frame is obtained by repeating a process of sequentially transmitting the imaging ultrasound in the respective scanlines and collecting data.

The image processing unit 130 generates an electrical signal for generating the imaging ultrasound and the treatment ultrasound, or converts a converted electrical signal from an echo signal into an image signal. Specifically, the image processing unit 130 receives synchronized ultrasound generating signals under the control of the synchronizing unit 140 and generates an ultrasound generating signal for each transducer element set by the direction of the ultrasound before inputting the generated signal to each corresponding transducer element. Further, the image processing unit 130 converts electrical signals generated by the transducer elements each receiving an ultrasound, into a single electrical signal, and converts the electrical signal into an image signal.

A method for generating an image picture by the image processing unit 130 is as follows. A target region in an ultrasound image is divided into a plurality of scanlines according to the transmission directions of imaging ultrasounds. The imaging transducer 120 operates to transmit an imaging ultrasound for each of the scanlines. The imaging transducer 120 receives an echo signal of the imaging ultrasound it transmitted. At this time, each scanline brightness image (B-mode image) is obtained with its location specified based on the time between the transmission time and the reception time. The imaging transducer 120 repeats this process, to acquire images of a plurality of scanlines. The image processing unit 130 forms one frame by displaying the plurality of scanlines, thus generating an image picture. Therefore, the location of an object appearing on the image picture is generally determined by the time between the transmission time of the imaging ultrasound by the imaging transducer 120 and the reception time of the reflected imaging ultrasound by the imaging transducer 120. Determining the location of an object appearing on the image picture is supposed to be performed based normally on the time required to receive reflected imaging ultrasound by the imaging transducer 120 after the imaging ultrasound is reflected at the object. However, this can be replaced by halving the time required for the imaging transducer 120 to generate the imaging ultrasound and receive the reflected imaging ultrasound, and hence the time between the transmission and reception times serves as the basis for displaying the picture.

A process is described below for synchronizing times at which the imaging ultrasound and the high-intensity ultrasound reach a specific location (focal point) by the synchronizing unit 140. The synchronizing by the synchronizing unit 140 equalizes the time for the imaging ultrasound to be reflected at the object and for reflected echo signal thereof to be received by the transducer to that of the high-intensity ultrasound to be reflected and its reflected echo signal to be received. Even if the pictures of the ultrasounds generated by the treatment transducer 110 and the imaging transducer 120 are displayed at once, coordinates of a specific location (focal point) coincide when displayed.

The synchronizing unit 140 synchronizes the ultrasound generation times of the treatment transducer 110 and the imaging transducer 120. Specifically, the synchronizing unit 140 receives an input of a region of focusing the high-intensity ultrasound generated by the treatment transducer 110, from the input unit 170 or the control unit 150. The synchronizing unit 140 controls (synchronizes) the transducer unit 125 such that the imaging ultrasound and the high-intensity ultrasound simultaneously reach this focal point. The synchronization mentioned here means to synchronize the ultrasound generation times by calculating traveling times of the ultrasounds based on a distance between each of the transducers and the focal point. A synchronization method can be determined differently for each scanline. In some embodiments, the synchronizing unit 140 generates synchronized signals with their ultrasound generation times adjusted to generate the ultrasound by the imaging transducer 120 through scanning the region after the synchronization at a preset focal point if crossed by the scanline or by its preset range that is crossed by the scanline while scanning other regions without synchronization or skipping an ultrasound generation by the treatment transducer 110.

The ultrasound medical apparatus 100 according to some embodiments is useful because it obviates the need for any special process but an operation of the control unit 150 in obtaining an image having a typical ultrasound image with the focal point of the high-intensity ultrasound co-displayed. In some embodiments, the synchronizing unit 140 is illustrated as a separate block although it represents one of functional sections of the control unit 150. However, the present disclosure covers the synchronizing feature regardless of how the functionality of the synchronizing unit 140 is implemented.

The synchronizing unit 140 establishes virtual channels 610 respectively for scanlines of transducer elements of the imaging transducer 120, and determines the transmission times of the treatment channels 620 based on the locations of the virtual channels 610 and respective channels of the treatment transducers 110 and the focal point location. Hereinafter, the virtual channels 610 and the respective channels of the treatment transducers 110 are collectively referred to as "treatment channels 620." In some embodiments, the multiple virtual channels 610 may be replaced by at least one channel.

In some embodiments, the synchronizing unit 140 establishes the virtual channels 610 exclusively with the transducer element corresponding to a scanline that passes through the focal point among the transducer elements of the imaging transducer 120.

In some embodiments, the synchronizing unit 140 establishes the virtual channels 610 exclusively with the transducer element corresponding to a scanline that is closest to the focal point among the transducer elements of the imaging transducer 120.

Based on an arrival time information ($T_i$) required for ultrasounds of the imaging ultrasound and high-intensity ultrasound transmitted through the treatment channels 620 to reach the focal point and on a maximum arrival time information ($Max(T_i)$) among a plurality of pieces of the arrival time information ($T_i$), the synchronizing unit 140 calculates a delay time information ($T_i$) for each of the treatment channels 620 and determines the transmission times of the treatment channels 620 based on the respective delay time information ($T_i$).

The synchronizing unit 140 calculates the delay time information ($T_i$) respectively for the treatment channels 620 by subtracting the respective arrival time information ($T_i$) from the maximum arrival time information ($Max(T_i)$).

By applying the respective delay time information ($T_i$) to the transmission times in the treatment channels 620, which correspond to the maximum arrival time information ($Max(T_i)$), the synchronizing unit 140 determines the transmission times in the other treatment channels 620.

The synchronizing unit 140 calculates the respective arrival time information ($T_i$) based on traveling speed information ($C$) at which the imaging ultrasound and the high-intensity ultrasound travel in a medium of the target object, focal-point coordinate values ($f_x$, $f_y$, $f_z$) corresponding to the focal point, and channel coordinate values ($e_{x\_i}$, $e_{y\_i}$, $e_{z\_i}$) corresponding to the treatment channels 620.

The synchronizing unit 140 calculates a distance difference information between the focal-point coordinate values ($f_x$, $f_y$, $f_z$) and the channel coordinate values ($e_{x\_i}$, $e_{y\_i}$, $e_{z\_i}$) on a rectangular coordinate system, and takes values obtained by dividing the distance difference information by the traveling speed information ($C$) as the respective arrival time information ($T_i$).

The synchronizing unit 140 takes coordinates of a transducer element located at a center among the transducer elements constituting the virtual channels 610 as the channel coordinate values of the virtual channels 610.

The synchronizing unit 140 repeats a process of establishing a virtual channel 610 in a manner that a partial image picture for a single scanline is formed in one cycle of the high-intensity ultrasound until a frame of the image pictures is formed.

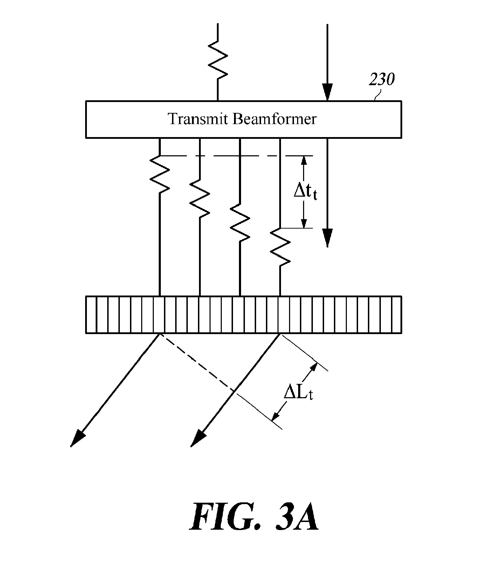

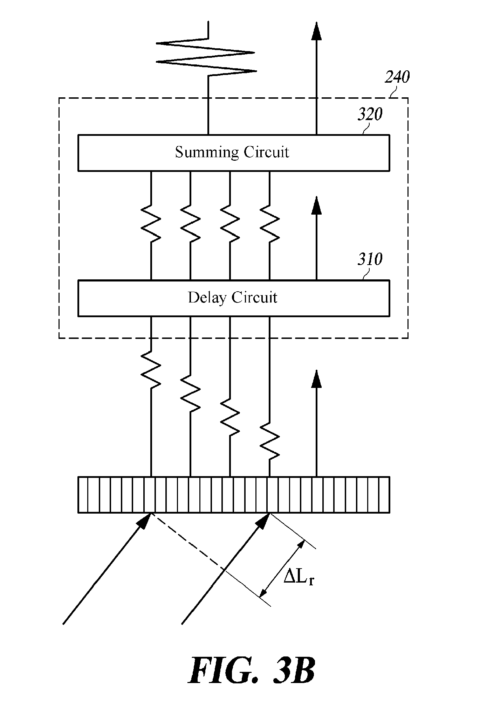

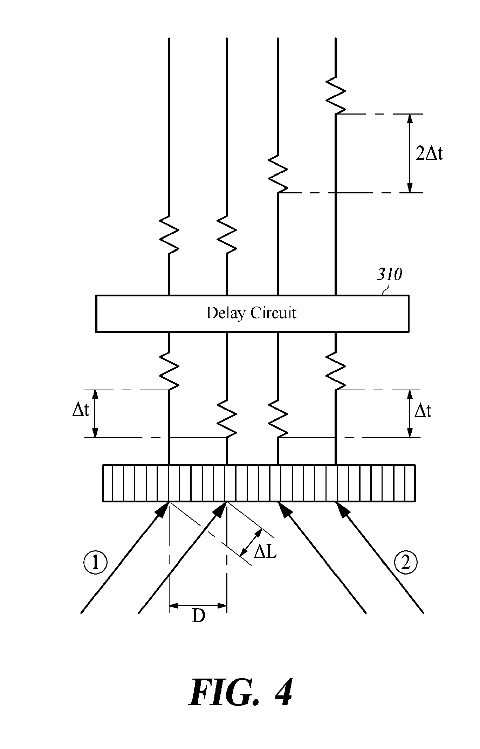

One of the features in some embodiments is that the synchronizing unit 140 synchronizes the ultrasound generation timings of the treatment transducer 110 and the imaging transducer 120 via the delay time, thus displaying the focal point on the image picture. At this time, the high-intensity ultrasound echo signal can be irrelevant of a scanning direction of the imaging transducer 120 when it is received by the imaging transducer 120 from the focal point 510, which causes no problem in the present disclosure. Such directional irrelevancy is overcome by the imaging transducer 120 formed of a transducer array, wherein the high-intensity ultrasound echo signal at the focal point is displayed as a strong signal when the scanning direction is toward the focal point, and the high-intensity ultrasound echo signal at the focal point is displayed as a noise when the scanning is not directed to the focal point. This is described in detail later with reference to FIGs. 3 and 4.

The control unit 150 controls the overall functions of the ultrasound medical apparatus 100, receives information on the focal point of a user from the input unit 170, or re-adjusts the orientation of the treatment transducer 110 based on the image picture. The control unit 150 further stores the generated image picture in the storage unit 160, and outputs the stored image picture.

The display unit 180 displays an image signal generated by the image processing unit 130. The storage unit 160 stores the image picture generated by the image processing unit 130 or an adjustment value of each transducer corresponding to the specific focal point 510. The adjustment value of the transducer includes information on a steered angle of each transducer or the ultrasound generation time.

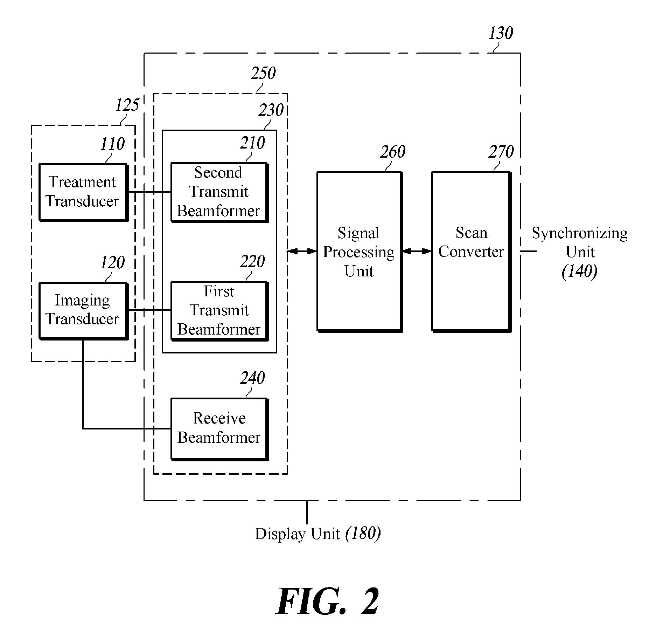

FIG. 2 is a schematic block diagram of an image processing unit according to some embodiments.

The image processing unit 130 includes a beamformer unit 250, a signal processing unit 260 and a scan converter 270. The beamformer unit 250 delays, in a transducer array including a plurality of transducer elements, an electrical signal appropriate for the corresponding transducer to convert an electrical signal into an appropriate electrical signal for each of the transducer elements, or it delays and sums electrical signals converted at the respective transducer elements, to calculate an output value of the corresponding transducer. The beamformer unit 250 includes a transmit beamformer 230 and a receive beamformer 240.

By adjusting a magnitude of the time delay of the electrical signal to be inputted to the transducer unit 125, the transmit beamformer 230 generates an electrical signal to be inputted to each transducer or the transducer elements of the transducer array. The transmit beamformer 230 includes a first transmit beamformer 210 and a second transmit beamformer 220.

The first transmit beamformer 210 is coupled to the treatment transducer 110. In some embodiments, the treatment transducer 110 is not formed of a transducer array, where the first transmit beamformer 210 inputs a single signal for generating an ultrasound at precise timing to the treatment transducer 110 under a control of the synchronizing unit 140. In some embodiments, the treatment transducer 110 is a transducer array, where the first transmit beamformer 210 is similar to the second transmit beamformer 220 in terms of function.

The second transmit beamformer 220 is coupled to the imaging transducer 120. In some embodiments, the imaging transducer 120 is a transducer array as described above, and the second transmit beamformer 220 divides and delays a single signal for generating an ultrasound at precise timing, generates an electrical signal appropriate for each transducer element, and inputs the electrical signal to the imaging transducer 120, under a control of the synchronizing unit 140.

The receive beamformer 240 aggregates the electrical signals received by transducer elements respectively, and converts those signals into a single electrical signal for use as an image signal. The transducer array as the imaging transducer 120 has its multiple transducer elements respectively converting the ultrasounds into electrical signals, and hence each electrical signal has a different form depending on the path of each ultrasound. The receive beamformer 240 utilizes a method for converting electrical signals to have a common phase through a time delay compensative for path differences between different signal paths and superimposing the compensated signals into a single electrical image signal. Details on the operations of the second transmit beamformer 220 and the receive beamformer 240 are described later with reference to FIGs. 3 and 4.

The signal processing unit 260 is a module for converting an echo signal into a desired signal. The electrical signal inputted from the imaging transducer 120 is a reflected wave of a pulsed wave, which is inputted as a form of wave. For a Doppler image, the electrical signal is converted into a signal calculated by the Doppler formula, and for a B-mode image, it is converted into a signal in which amplitude is converted into brightness.

The scan converter 270 converts the image picture into data of a format that is used in the display unit 180 of a predetermined scanline display system. The scan converter 270 stores images for observing and recording while transmission and reception of the imaging ultrasound is performed, and converts electrical signals received by each scanline into image data that can be displayed on the display unit 180.

FIG. 3 is a schematic diagram for illustrating a beam forming process by a transducer array according to some embodiments.

The transducer of the transducer unit 125 refers to a device for converting electrical signal into mechanical energy or for converting mechanical energy into electrical signal. The transducer of the transducer unit 125 is achieved by using a piezoelectric element, and hence both the functions of converting the electrical signal into the mechanical energy and converting the mechanical energy into the electrical signal can be achieved with a single element.

The transducer array of the transducer unit 125 refers to an array of transducer elements in which a plurality of transducer elements is tightly arranged. The transducer element is achieved by using a piezoelectric element, and takes a role of generating or receiving an ultrasound. The transducer elements of the transducer array are configured to respectively receive or generate different signals, and hence they operate a manner independent from each other. The transmit beamformer 230 controls the traveling direction of each ultrasound by applying a different time delay on an electrical signals for generating ultrasound for each of the transducer elements of the transducer array depending on the location of the transducer element.

In the example shown in FIG. 3A, the transducer array of the transducer unit 125 generates the ultrasound in the left direction. The electrical signal for generating the imaging ultrasound or the high-intensity ultrasound, which is synchronized by the synchronizing unit 140 is converted into a signal that is appropriately delayed depending on the location of the transducer element by the transmit beamformer 230. Each transducer element generates an ultrasound based on the received signal. As the transducer element can be considered as a point wave source by the Huygens' Principle, and hence a plane wave having a path difference by the delayed time of the signal is generated. When the speed of ultrasound in the same medium is C, a relation between a time delay $\Delta t_t$ and a path difference $\Delta l_t$ between ultrasound generation transducer elements shown in FIG. 3 is represented by Equation 1. In general, C is assumed to be constant regardless of the frequency, at 1540 m/s that is the sound speed in a soft tissue.

$$\Delta t_t = \frac{\Delta l_t}{C}$$

Equation 1

In other words, because there exists a path difference depending on the transmission direction and the location of the transducer element, when transmitting an ultrasound, the transducer array of the transducer unit 125 generates the ultrasound based on a signal obtained by delaying an ultrasound generating signal considering the path difference. Not only when generating the imaging ultrasound, but also when receiving an ultrasound, such a path difference exists depending on the reception direction. Accordingly, when receiving an ultrasound, the transducer array of the transducer unit 125 receives it as a signal with a constructive interference by time delaying the signal received by each transducer element depending on the path difference.

In the example shown in FIG. 3B, the transducer array of the transducer unit 125 receives an ultrasound from the left direction. The echo signal is converted into a signal that is appropriately delayed depending on the location of each transducer element and summed by the receive beamformer 240. Although the output image signal should be a single signal because the image is a single image, as the transducer array receives a number of ultrasounds having a path difference from each other depending on the locations of the transducer elements, a number of signals are generated. A delay circuit 310 delays and synchronizes each signal appropriately for the path difference. A summing circuit 320 composes the synchronized signals, to convert them into a single signal with the constructive interference.

In other words, as an incident angle of the reflected wave is determined by the transmission direction, the transducer array composes the input signals into a single signal with the constructive interference by applying a time delay corresponding to the incident angle.

In general, a directivity is not considered in receiving an ultrasound in an ultrasound apparatus, because one precludes a case where ultrasounds are simultaneously inputted from different directions from each other; however, as the time delay required for the constructive interference changes with the incident angle, an input wave inputted from an unexpected direction is not included in the constructive interference, and hence the reception of an ultrasound has the directivity.

The directivity employed in some embodiments refers to a phenomenon in which, particularly in converting the ultrasound into the electrical signal by the transducer array, an ultrasound received from a specific direction is dominantly converted into the electrical signal, and ultrasounds received from the other directions are detected as a noise.

FIG. 4 is a schematic diagram for illustrating a reception directivity of the transducer array according to some embodiments.

In FIG. 4, an electrical signal is shown, which is generated when another ultrasound is received from the right direction that is opposite to the reception direction of the ultrasound received in the process of transmitting the ultrasound and receiving the reflected wave in the left direction as shown in FIGs. 3A and 3B. Hereinafter, the left direction is referred to as direction ①, and the right direction is referred to as direction ②.

As described with reference to FIG. 3B, the ultrasounds received from direction ① are composed into a single signal and amplified, after converting signals received by the transducer elements on the opposite sides into the same signal by increasing the delay as the transducer element is located on the left side. On the contrary, in the case of the ultrasounds received from direction ②, although the transducer element on the left side generates a signal later than the transducer element on the right side, because the delay is increased as the transducer element is located on the left side, which is similar to the signal received from direction ①, the compensation for the path difference is decreased as the transducer element is located on the left side. Accordingly, the ultrasounds received from direction ② are not converted into the same signal by the time delay, and hence they are composed as distributed signals without the constructive interference. When the ultrasounds are inputted at the same angle with intervals of the same width (D), so that a path difference is generated by $\Delta L$, a time difference of $\Delta t$ is generated. Therefore, when the ultrasound is inputted from direction ①, signal inputted at the left side is delayed by $\Delta t$, to synchronize it with the same time/ however, when the ultrasound is inputted from direction ②, time intervals between the signals are increased by $2\Delta t$ with the same delay. Therefore, in the case of the transducer array, a signal of the incident angle that is compensated by the time delay is subjected to the constructive interference, and a signal of the incident angle that is not compensated by the time delay is spread on the time axis and detected as a noise.

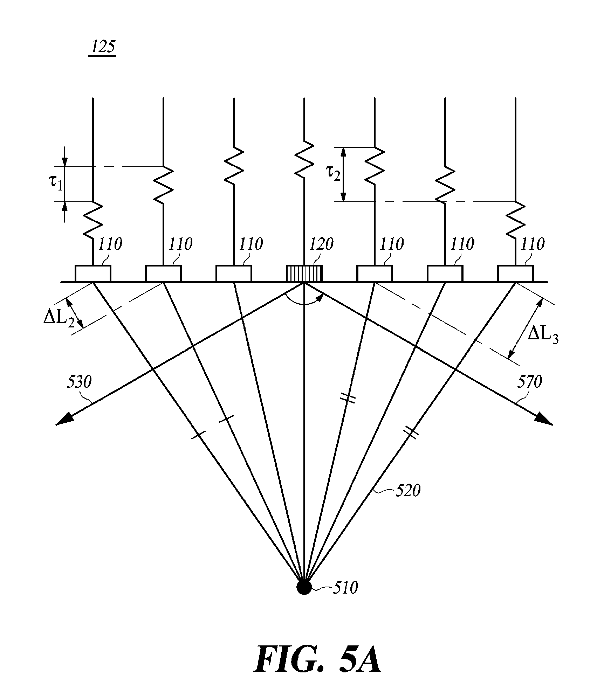

FIG. 5A is a schematic diagram for illustrating the transducer unit generating an ultrasound under a control of the synchronizing unit according to some embodiments.

The transducer unit 125 includes the treatment transducer 110 and the imaging transducer 120 as mentioned in the description of FIG. 1. The imaging transducer 120 scans an image of a predetermined region, and hence it scans for each scanline from one end line 530 to the other end line 570 with reference to the point where the imaging transducer 120 is located.

The treatment transducer 110 generates an ultrasound with a treatment position as the focal point 510. As it is hard to acquire an image by the imaging transducer 120 if the ultrasound is continuously generated, the treatment transducer 110 periodically generates a pulse signal. At this time, a path difference occurs in the distance to the focal point 510 depending on the location of the treatment transducer 110. In order to make sure that the high-intensity ultrasound simultaneously reaches the focal point 510, this path difference should be compensated. The path difference and a time interval for generating the ultrasound by compensating for the path difference are shown in the figure. An ultrasound path at the treatment transducer 110 that is farthest from the focal point 510 and an ultrasound path at another treatment transducer 110 are selected. The path difference can be obtained by subtracting a distance between the focal point 510 and another treatment transducer 110 from a distance between the focal point 510 and the treatment transducer 110 that is farthest from the focal point 510. Considering this path difference, a delayed ultrasound is generated from another treatment transducer 110. By adjusting the time for generating the ultrasound at the treatment transducer 110 in this manner, the ultrasounds generated from the treatment transducer 110 simultaneously reach the focal point 510. In a similar manner, the time for generating the ultrasound is adjusted for the imaging transducer 120. The high-intensity ultrasound and the ultrasound generated by the imaging transducer 120 have the same speed in the same medium, and hence the same delay is applied to make the ultrasounds reach the focal point 510 at the same time. In this manner, the times for the ultrasound generated by the treatment transducer 110 and reflected at the focal point 510 and the ultrasound generated by the imaging transducer 120 and reflected at the focal point 510 to reach the imaging transducer 120 are adjusted to be same as that for each other.

FIG. 5A is a schematic diagram of the transducer unit 125 in which the imaging transducer 120 is arranged at the center and the treatment transducer 110 is arranged on both sides in a line-symmetric manner with respect to the imaging transducer 120. When the focal point is set below the imaging transducer 120, the treatment transducer 110 located at each of both ends is the treatment transducer 110 that is farthest from the focal point 510. The synchronizing unit 140 controls the beamformer unit 250 based on this configuration, to adjust the ultrasound generation times of the treatment transducer 110 and the imaging transducer 120. Any transducer can be the basis for adjusting the ultrasound generation time; however, as the beamformer employs a delay circuit, a method of determining the delay amount based on the transducer that is farthest from the focal point, which first generates the ultrasound, is described below. Describing the method of determining the delay amount of a neighboring transducer based on the transducer that is farthest from the focal point, a transducer having a distance from the focal point shorter than that of the transducer that is farthest from the focal point 510 has a path difference by a difference between the distances to the focal point. The ultrasound needs to be transmitted with a time delay corresponding to the time for the ultrasound to travel the corresponding path difference to make the arrival times equal to each other, and hence a delay time T for delaying the ultrasound generation time of the corresponding transducer is set by dividing the path difference calculated for each transducer by the speed of the ultrasound. This is an example of how to calculate the delay time information based on the length of the path difference.

The delay time information is described below based on the time required for the ultrasound reach the focal point 510 from each transducer.

The closest transducer has a distance from the focal point 510 shorter than that of the farthest transducer, and hence it should generate the ultrasound later in time than the farthest transducer. The delay time information T is calculated by dividing this path difference by the traveling speed information C of the imaging ultrasound and the high-intensity ultrasound.

In some embodiments, the ultrasound generation delay time information T is calculated in a manner that, based on a transducer having the longest time for the ultrasound to reach the focal point from the transducer (i.e., the farthest transducer) determined by calculating the time for the ultrasound to reach the focal point from each transducer, times at which the ultrasounds generated by the rest of the transducers respectively reach the focal point become equal to each other.

A time $T_i$ required for the ultrasound to reach the focal point 510 from the ith transducer can be obtained by calculating a straight distance between the focal point 510 and the ith transducer based on three-dimensional coordinates of the focal point 510 and the transducer and dividing the straight distance by the speed of the ultrasound, as represented by Equation 2.

$$T_i = \frac{1}{C} \times \sqrt{(f_x - e_{x\_i})^2 + (f_y - e_{y\_i})^2 + (f_z - e_{z\_i})^2}$$

Equation 2

In Equation 2, $T_i$ is time required for the ultrasound to reach the focal point 510 from the ith transducer (hereinafter, "arrival time information"), C is traveling speed information of the imaging ultrasound and the high-intensity ultrasound in the medium, $f_x$ is x-coordinate of the coordinates of the focal point, $f_y$ is y-coordinate of the coordinates of the focal point, $f_z$ is z-coordinate of the coordinates of the focal point, $e_{x\_i}$ is x-coordinate of the coordinates of the ith treatment channel, $e_{y\_i}$ is y-coordinate of the coordinates of the ith treatment channel, and $e_{z\_i}$ is z-coordinate of the coordinates of the ith treatment channel. In this case, the three-dimensional coordinates are represented as coordinates on the rectangular coordinate system.

Now that the time for the ultrasound to reach the focal point 510 from each transducer is obtained, the ultrasound generation time synchronized for the ultrasound to simultaneously reach the focal point 510 for each transducer can be represented in a form of being delayed by the delay time information T from the ultrasound generation time of the transducer that is farthest from the focal point 510.

The delay time information T for each transducer is represented by Equation 3.

$$\tau_i = Max(T_i) - T_i$$

Equation 3

In Equation 3, $T_i$ is relative delay time of the ultrasound generation time of the ith transducer from the ultrasound generation time of the farthest transducer, $Max(T_i)$ is maximum arrival time information indicating the largest value among values of $T_i$ calculated by Equation 2, $T_i$ is arrival time information required for the ultrasound to reach the focal point 510 from the ith transducer.

In this manner, the synchronizing unit 140 controls the beamformer unit 250 by calculating the delay time information T for each transducer, to generate the ultrasound.

The imaging transducer 120 is not necessarily located at the center, each transducer is not necessarily arranged on the same plane, and the treatment transducer 110 is not necessarily arranged in the symmetric manner. So long as each transducer is arranged in a synchronized manner under the control of the synchronizing unit 140, such that the imaging ultrasound and the high-intensity ultrasound simultaneously reach the focal point 510, which is the main feature in some embodiments, any mode is included in the scope of the present disclosure.

In the above description, the imaging transducer 120 is synchronized with the high-intensity ultrasound to scan a single scanline. The imaging transducer 120 forms a single frame by scanning all scanlines corresponding to the single frame, and hence an image of a single frame can be obtained by repeating the process of transmitting and receiving the synchronized ultrasound while changing the scanlines.

A scanline that is closest to the focal point 510 receives the echo signal of the high-intensity ultrasound reflected at a location that is closest to the focal point 510; however, a scanline that is far from the focal point 510 detects the echo signal of the high-intensity ultrasound reflected at a location near the focal point 510 as a noise due to the directivity. Therefore, a single frame of an image picture can be obtained by repeating a process in which the imaging transducer 120 corresponding to a scanline that passes the focal point 510 or a scanline that passes vicinity of the focal point 510 performs a scanning by transmitting and receiving the imaging ultrasound synchronized with the treatment transducer 110 and receiving the high-intensity ultrasound, and the imaging transducer 120 corresponding to a scanline that is deviated from the focal point 510 performs a scanning by transmitting and receiving an ultrasound that is not synchronized with the treatment transducer 110. The process of performing a scanning by transmitting and receiving an ultrasound (imaging ultrasound and high-intensity ultrasound) that is not synchronized includes a process of scanning while the treatment transducer 110 does not operate.

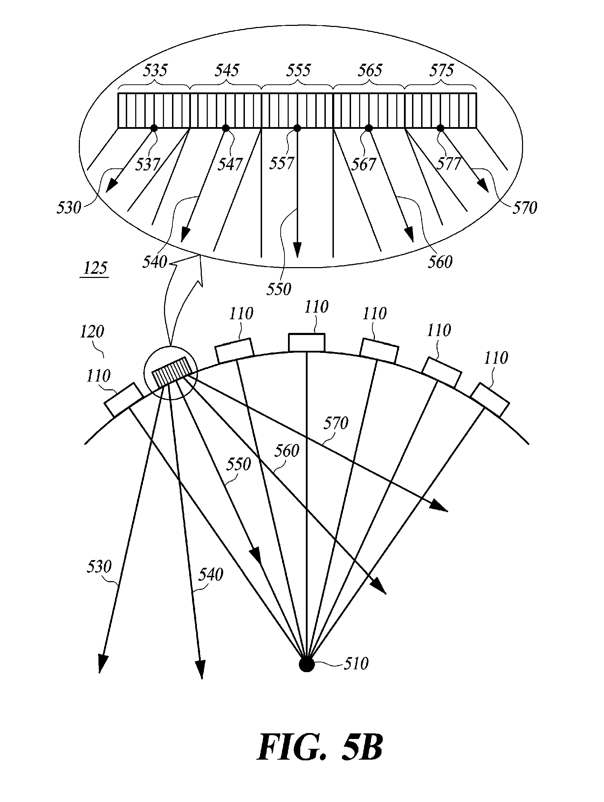

FIG. 5B is a schematic diagram of the transducer unit 125 of a circular focusing type according to some embodiments.

When the transducers are arranged on a surface of a sphere and the center of the sphere is set as the focal point, all the transducers have the same distance to the focal point 510, and hence all the transducers are controlled to generate the ultrasound at the same time. The direction of the imaging transducer 120 is continuously changed as it sequentially scans a predetermined region, and the treatment transducer 110 generates the ultrasound in a fixed direction to the focal point 510.

In some embodiments, the imaging transducer 120 is configured as the transducer array as described above. In some embodiments, the imaging transducer 120 performs a scanning by using a specific transducer element set for each scanline. A transducer array is shown in FIG. 5B, which includes a first transducer element set 535 corresponding to a first scanline 530, a second transducer element set 545 corresponding to a second scanline 540, a third transducer element set 555 corresponding to a third scanline 550, a fourth transducer element set 565 corresponding to a fourth scanline 560, and a fifth transducer element set 575 corresponding to a fifth scanline 570.

Each of the transducer element sets operates as if it is an independent imaging transducer 120 in a sense of creating an image picture corresponding to the scanline, and only a neighboring transducer element set operates at a single scan timing among the transducer element sets. As shown in FIG. 5A, a transducer element set corresponding to a scanline that passes the focal point 510 or a scanline that passes vicinity of the focal point 510 should transmit the imaging ultrasound at the time synchronized with the treatment transducer 110, and a location of the transducer element set should be specified to perform the synchronization. It is because the synchronizing unit 140 can calculate the path difference of the treatment transducer 110 and the transducer element set with respect to the focal point 510 only when the location is specified.

As each of the transducer element sets operate as if it is an independent imaging transducer 120, the location of each of the transducer element sets is defined by the center point of the transducer element set. Therefore, the location of the first transducer element set 535 is defined by a first transducer element center point 537, the location of the second transducer element set 545 is defined by a second transducer element center point 547, the location of the third transducer element set 555 is defined by a third transducer element center point 557, the location of the fourth transducer element set 565 is defined by a fourth transducer element center point 567, and the location of the fifth transducer element set 575 is defined by a fifth transducer element center point 577. The synchronization is performed by taking this location as the coordinates of the treatment channel in Equation 2.

In this case, as shown in FIG. 5B, the scanline that passes the focal point 510 can synchronize the third scanline 550 or the second and fourth scanlines 540 and 560 that are relatively close to the focal point 510.

In the transducer array, the number of scanlines is determined according to the resolution, and hence it is typical to include 128 or more scanlines; however, in the example shown in FIG. 5B, it is assumed to include five scanlines for the sake of explanation. The number of scanlines or the number of transducer element sets is not limited to the example shown in FIG. 5B in practice, but any number larger than five can be applied.

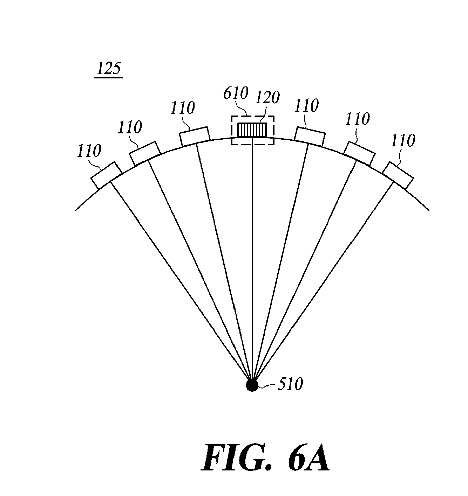

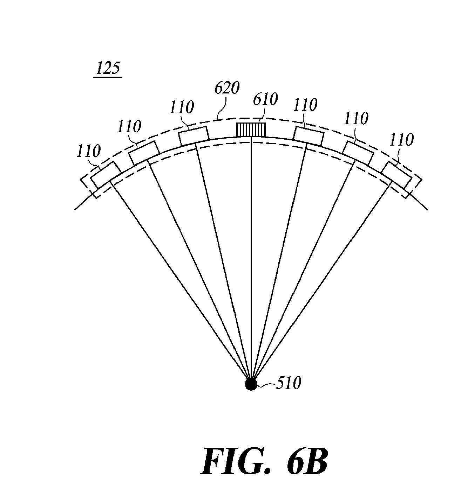

FIGs. 6A and 6B are schematic diagrams of a virtual channel and a treatment channel according to some embodiments.

FIG. 6A is a schematic diagram for illustrating a virtual channel 610 in the sphere focusing type transducer unit according to some embodiments. In some embodiments, the virtual channel 610 includes a plurality of channels (at least one or more channel); however, the present disclosure is not limited to this scheme, but in some embodiments, the virtual channel 610 includes a single channel.

As a synchronized ultrasound should be generated when the imaging transducer 120 generates the imaging ultrasound, the ultrasound generation time should be specified.

The synchronizing unit 140 recognizes the virtual channel 610 for each scanline of the imaging transducer 120.

FIG. 6B is a schematic diagram for illustrating a treatment channel in the sphere focusing type transducer unit according to some embodiments. The treatment transducer 110 is controlled in a manner that the high-intensity ultrasounds simultaneously reach the focal point by the synchronizing unit 140, and each control target is referred to as a treatment channel 620. The virtual channel 610 described with reference to FIG. 6A is recognized as a sort of the treatment channel 620, and the arrival times of the imaging ultrasound and the high-intensity ultrasound are synchronized based on information on distance differences between the focal point and the location of the treatment channel as if the treatment channel 620 is controlled. With this method as well, the same effect as that of the configuration and the method described with reference to FIG. 5A can be obtained.

In other words, the virtual channel 610 is a channel that is included in the treatment channel 620 for focusing the high-intensity ultrasound. While the treatment channel 620 is a channel for controlling a number of high-intensity ultrasounds to reach the focal point 510 at the same time and focusing the high-intensity ultrasounds, the virtual channel 610 is a channel for controlling the imaging ultrasound to reach the focal point 510 at the same time with the high-intensity ultrasound, such that the reflected wave of the high-intensity ultrasound reflected at the focal point 510 appears at the precise location on the image picture.

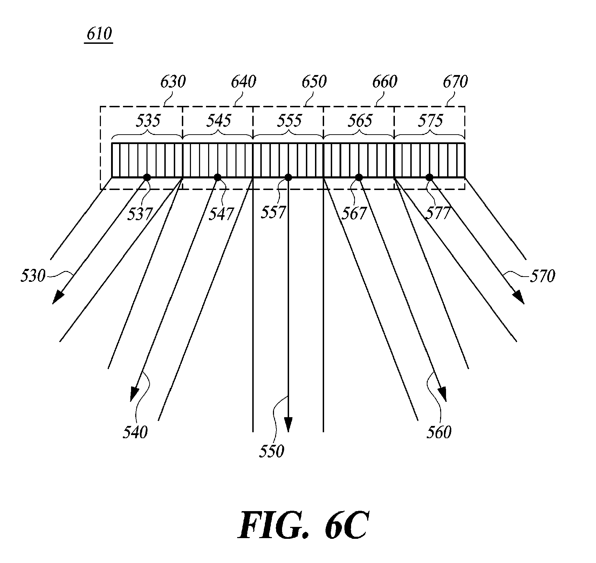

FIG. 6C is a schematic diagram for illustrating the virtual channel for each transducer element set according to some embodiments.

As described with reference to FIG. 5B, in some embodiments the imaging transducer 120 is a transducer array, and the transducer array can perform a scanning by designating a transducer element set for each scanline. In the example shown in FIG. 6C, the imaging transducer 120 with the transducer element set formed with respect to five scanlines as shown in FIG. 5B is described as an example.

With respect to the virtual channel 610, when the imaging transducer 120 is divided into a plurality of transducer element sets, the virtual channel for determining the transmission time of the imaging ultrasound should be defined in a separate manner, because each transducer element set has a different time for transmitting the imaging ultrasound. Therefore, virtual channels corresponding to the first transducer element set 535 to the fifth transducer element set 575 can be separately set as first to fifth virtual channels 630 to 670, respectively. In this case, as shown in FIG. 5B, coordinates of the virtual channels 630 to 670 required for the synchronization of each of the virtual channels become the center points 537 to 577 of the transducer elements, respectively.

In this case as well, it can be determined whether or not to perform the synchronization based on the scanline that passes the focal point 510 or is closest to the focal point 510.

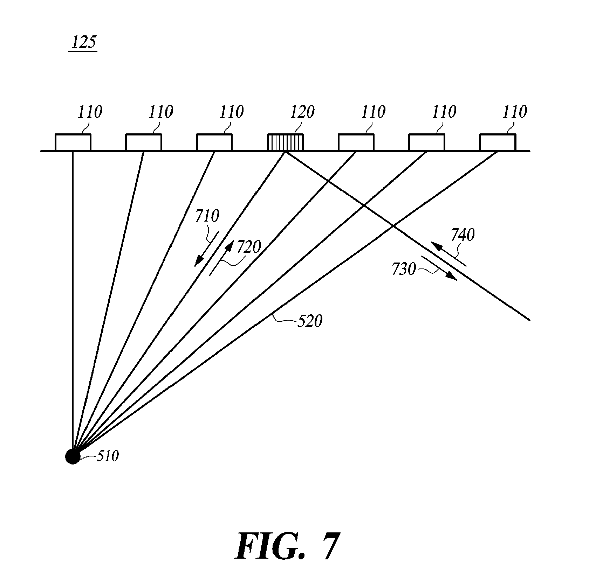

FIG. 7 is a schematic diagram for illustrating an actual application of the reception directivity according to some embodiments.

When the imaging transducer 120 generates an ultrasound 730, in a direction other than the focal point 510, a reflected wave 740 is reflected back from the direction different from the focal point 510. However, as the treatment transducer 110 also generates an ultrasound, a reflected wave 720 is also reflected back from the focal point 510. In this case, the incident angles are different from each other. As described with reference to FIG. 3, in the transducer array, the receive beamformer 240 delays each signal such that only the ultrasound received from the direction of transmitting the ultrasound is subjected to the constructive interference, and hence the reflected wave from the focal point 510 is not received with the constructive interference when generating the ultrasound 730 in the direction different from the focal point 510 but is detected as a spread noise. On the other hand, when the imaging transducer 120 generates an ultrasound 710 in the direction of the focal point 510, the reflected wave of the ultrasound 710 in the direction of the focal point 510 and the reflected wave of the high-intensity ultrasound are reflected together at the same time from the focal point 510. Therefore, the reflected wave 720 from the focal point 510 is received as a signal with the constructive interference, and as this signal has the same path as the reflected wave of the ultrasound 710 in the direction of the focal point 510, it is displayed at the exact location on the image picture.

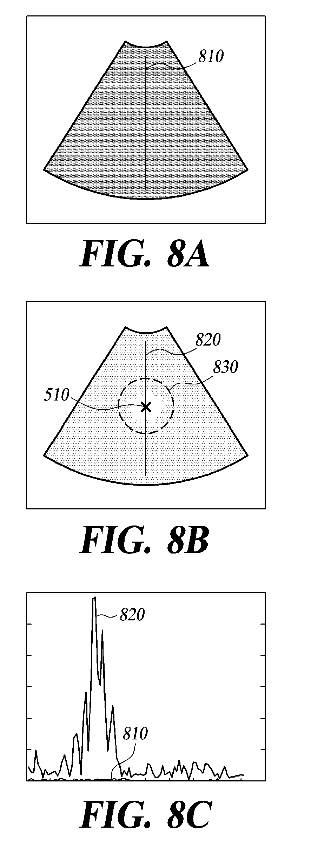

FIG. 8A is a schematic diagram of an image picture created while the high-intensity ultrasound is not generated.

A frame of an image picture includes a plurality of scanlines. A scanline that passes the focal point 510 set on the treatment transducer 110 among the plurality of scanlines is displayed as a control group scanline 810 on the image picture.

FIG. 8B is a schematic diagram of an image picture created while the high-intensity ultrasound is generated.

In FIG. 8B, a scanline of the high-intensity ultrasound that passes the focal point 510 is displayed as an experimental group scanline 820. The experimental group scanline 820 is displayed to correspond to the control group scanline 810 that passes the focal point 510 set on the treatment transducer 110 shown in FIG. 8A.

As no particular echo signal is detected at the focal point 510 in FIG. 8A, the focal point 510 is displayed in black on the control group scanline 810 that passes the focal point 510 set on the treatment transducer 110. In contrast, as a strong echo signal is detected at a bright area 830 in FIG. 8B, the focal point 510 is displayed in white on the experimental group scanline 820 of the high-intensity ultrasound that passes the focal point 510. The focal point 510 can be confirmed in this manner.

FIG. 8C is a graph showing the intensity of the ultrasound received by the imaging transducer at a scanline that passes the focal point, depending on whether or not the high-intensity ultrasound is generated.

When the treatment transducer 110 transmits no high-intensity ultrasound, no particular change appears at the focal point 510 on the control group scanline 810 that passes the focal point 510 set on the treatment transducer 110. When the treatment transducer 110 generates the high-intensity ultrasound, which is then focused at the focal point, so that the echo signal is generated, an echo signal of a high amplitude is detected at the focal point 510 on the experimental group scanline 820 of the high-intensity ultrasound that passes the focal point 510. Accordingly, the focal point can be confirmed on the ultrasound image.

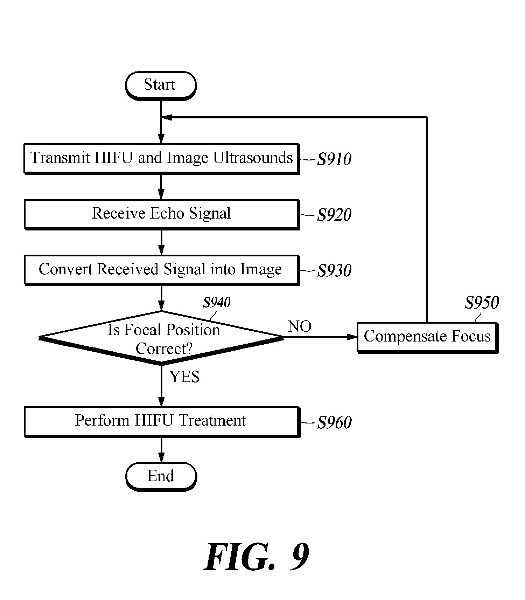

FIG. 9 is a flowchart of a method of confirming a focal point of a high-intensity ultrasound, according to some embodiments.

The imaging ultrasound and the high-intensity ultrasound are transmitted (step S910). The high-intensity ultrasound is transmitted in a form of pulse that is repeated with a focus adjusted on a treatment site, and the ultrasound generation time is adjusted such that the high-intensity ultrasound simultaneously reaches the focal point 510. The imaging ultrasound is synchronized in a manner that the ultrasound is generated at a time for reaching the focal point 510 simultaneously with the high-intensity ultrasound when the ultrasound is generated in the direction of the focal point 510. Although the treatment transducer 110 generates the ultrasound in a fixed direction unless the focal point 510 is re-adjusted, the imaging transducer 120 changes its direction in order to scan a predetermined region. Although the high-intensity ultrasound is configured to supply a high energy to destroy a tissue of the treatment site, at Step S910 for transmitting the high-intensity ultrasound to confirm the focal point, the high-intensity ultrasound can be generated with an intensity that does not affect the tissue. When confirming the focal point in the middle of a treatment, a continuous high-intensity ultrasound can be transmitted in a form of pulse.

The imaging transducer 120 receives the reflected signal (step S920). The imaging transducer 120 receives the reflected wave of the high-intensity ultrasound as well as the reflected wave of the ultrasound transmitted from it. In this case, the imaging transducer 120 uses the receive beamforming method to compensate for the path difference exclusively for the reflected wave in the transmission direction, and hence the ultrasound received from the direction to which the ultrasound is not transmitted is detected as a noise. The echo signal of the high-intensity ultrasound reflected at the focal point 510 is received as a clear signal (peak) only when the imaging transducer 120 has transmitted the ultrasound in the direction of the focal point.

The received signal is converted into a B-mode image (step S930). That is, the received signal is converted into a B-mode image in which amplitude of the reflected signal is converted into brightness. The imaging transducer 120 receives the reflected wave for a single direction at a time, and hence it collects reflected signals of a plurality of directions, to create an image of a single frame. The B-mode image can be displayed on a display.

It is confirmed whether or not the focal point 510 is correct (step S940). In other words, it is confirmed whether or not the focal point matches the target location on the affected area. As a region where a reflected wave higher than the transmitted wave is detected on the image picture is a region where the reflected wave of the high-intensity ultrasound is concentrated, the focal point 510 of the high-intensity ultrasound can be found. Accordingly, it can be confirmed whether or not the focal point 510 matches the target location of the affected area, and when the focal point 510 does not match the target location, the focal point 510 can be compensated (step S950).

When the focal point 510 is correct, the high-intensity ultrasound treatment is continued (step S960). When it is determined that the focal point 510 is correct, the intensity of the high-intensity ultrasound is increased to start the actual treatment or the high-intensity ultrasound is switched to a continuous wave to continue the treatment.

Although exemplary embodiments of the present disclosure have been described for illustrative purposes, those skilled in the art will appreciate that various modifications, additions and substitutions are possible, without departing from the spirit and scope of the claimed disclosure. Therefore, exemplary embodiments of the present disclosure have been described for the sake of brevity and clarity. Accordingly, one of ordinary skill would understand the scope of the claimed disclosure is not to be limited by the explicitly described above embodiments but by the claims and equivalents thereof.

CROSS-REFERENCE TO RELATED APPLICATION

If applicable, this application claims priority under 35 U.S.C §119(a) of Patent Application No. 10-2013-0021974, filed on February 28, 2013 in Korea, the entire content of which is incorporated herein by reference. In addition, this non-provisional application claims priority in countries, other than the U.S., with the same reason based on the Korean patent application, the entire content of which is hereby incorporated by reference.

**Claims**

1. An ultrasound medical apparatus, comprising:

    an imaging transducer including a plurality of transducer elements, each configured to transmit an imaging ultrasound to a target object;
    a plurality of treatment transducers, each configured to transmit a high-intensity ultrasound to a focal point corresponding to a specific focal-point information of the target object;
    a synchronizing unit configured to synchronize transmission times of the imaging transducer and the treatment transducers to render the imaging ultrasound and the high-intensity ultrasound to reach the focal point at the same time; and
    an image processing unit configured, after the imaging ultrasound and the high-intensity ultrasound are transmitted in a synchronized manner, to generate an image picture based on an echo signal received by the imaging transducer.

2. The ultrasound medical apparatus according to claim 1, wherein the synchronizing unit is configured:

to establish a virtual channel for the high-intensity ultrasound by each scanline for the transducer elements of the imaging transducer, and
to determine the transmission times of transmission channels for the treatment transducers based on a location of each of virtual channels and channels for the treatment transducers and the focal point, the virtual channels and the channels for the treatment transducers being collectively designated as treatment channels.

3. The ultrasound medical apparatus according to claim 2, wherein the synchronizing unit is configured to establish the virtual channels exclusively for a transducer element corresponding to a scanline that passes the focal point among the transducer elements of the imaging transducer.

4. The ultrasound medical apparatus according to claim 2, wherein the synchronizing unit is configured to establish the virtual channels exclusively for a transducer element corresponding to a scanline that is closest to the focal point among the transducer elements of the imaging transducer.

5. The ultrasound medical apparatus according to claim 2, wherein the synchronizing unit is configured:

to calculate a delay time information $(T_i)$ for each of the treatment channels based on arrival time information $(T_i)$ required for an ultrasound transmitted through the treatment channels to reach the focal point and maximum arrival time information $(Max(T_i))$ among a plurality of pieces of arrival time information and
to determine the transmission times of the treatment channels based on the delay time information $(T_i)$.

6. The ultrasound medical apparatus according to claim 5, wherein the synchronizing unit is configured to calculate the delay time information $(T_i)$ by subtracting the arrival time information $(T_i)$ from the maximum arrival time information $(Max(T_i))$.

7. The ultrasound medical apparatus according to claim 5, wherein the synchronizing unit is configured to apply the delay time information $(T_i)$ on the transmission times of the treatment channels corresponding to the maximum arrival time information $(Max(T_i))$ among the treatment channels to determine the transmission times of the rest of the treatment channels.

8. The ultrasound medical apparatus according to claim 5, wherein the synchronizing unit is configured to calculate the arrival time information $(T_i)$ based on traveling speed information (C) at which the imaging ultrasound and the high-intensity ultrasound travel in a medium of the target object, focal-point coordinate values $(f_x, f_y, f_z)$ corresponding to the focal point, and channel coordinate values $(e_{x\_i}, e_{y\_i}, e_{z\_i})$ corresponding to the treatment channels.

9. The ultrasound medical apparatus according to claim 8, wherein the synchronizing unit is configured:

to calculate distance difference information between the focal-point coordinate values $(f_x, f_y, f_z)$ and the channel coordinate values $(e_{x\_i}, e_{y\_i}, e_{z\_i})$ on a rectangular coordinate system, and
to take a value obtained by dividing the distance difference information by the traveling speed information (C) as the arrival time information $(T_i)$.

10. The ultrasound medical apparatus according to claim 8, wherein the synchronizing unit is configured to take coordinates of a transducer element located at a center among the transducer elements constituting the virtual channels as the channel coordinate values of the virtual channels.

11. The ultrasound medical apparatus according to claim 2, wherein the synchronizing unit is configured to repeat a process of establishing a virtual channel in a manner that a part of the image picture for a single scanline is formed in one cycle of the high-intensity ultrasound until the image picture of one frame is formed.

12. A method for confirming a focal point on an image picture in an ultrasound medical apparatus including an imaging transducer including a plurality of transducer elements each configured to transmit an imaging ultrasound and a plurality of treatment transducers each configured to transmit a high-intensity ultrasound, the method comprising:

synchronizing transmission times of the imaging transducer and the treatment transducers to render the imaging ultrasound and the high-intensity ultrasound to reach the focal point at the same time; and
generating, after the imaging ultrasound and the high-intensity ultrasound are transmitted in a synchronized manner, the image picture based on an echo signal received by the imaging transducer.

13. The method according to claim 12, wherein the synchronizing includes
establishing a virtual channel for the high-intensity ultrasound by each scanline for the transducer elements of the imaging transducer, and
determining the transmission times of transmission channels for the treatment transducers based on a location of each of virtual channels and channels for the treatment transducers and the focal point, the virtual channels and the channels for the treatment transducers being collectively designated as treatment channels.

14. The method according to claim 13, wherein the synchronizing includes establishing the virtual channels exclusively for a transducer element corresponding to a scanline that passes the focal point among the transducer elements of the imaging transducer.

15. The method according to claim 13, wherein the synchronizing includes establishing the virtual channels exclusively for a transducer element corresponding to a scanline that is closest to the focal point among the transducer elements of the imaging transducer.

16. The method according to claim 13, wherein the synchronizing includes
calculating a delay time information ($T_i$) for each of the treatment channels based on arrival time information ($T_i$) required for an ultrasound transmitted through the treatment channels to reach the focal point and maximum arrival time information ($Max(T_i)$) among a plurality of pieces of arrival time information and
determining the transmission times of the treatment channels based on the delay time information ($T_i$).

17. The method according to claim 16, wherein the synchronizing includes calculating the arrival time information ($T_i$) based on traveling speed information (C) at which the imaging ultrasound and the high-intensity ultrasound travel in a medium of the target object, focal-point coordinate values ($f_x$, $f_y$, $f_z$) corresponding to the focal point, and channel coordinate values ($e_{x\_i}$, $e_{y\_i}$, $e_{z\_i}$) corresponding to the treatment channels.

18. The method according to claim 17, wherein the synchronizing includes
calculating distance difference information between the focal-point coordinate values ($f_x$, $f_y$, $f_z$) and the channel coordinate values ($e_{x\_i}$, $e_{y\_i}$, $e_{z\_i}$) on a rectangular coordinate system, and
taking a value obtained by dividing the distance difference information by the traveling speed information (C) as the arrival time information ($T_i$).

*100*

*125*

*110*

Treatment Transducer

*120*

Imaging Transducer

*130*

Image Processing Unit

*140*

Synchronizing Unit

*150*

Controlling Unit

*170*

Input Unit

*160*

Storage Unit

*180*

Display Unit

# FIG. 1

*130*

*250*

*125*

*230*

*110*

*210*

| Treatment Transducer | Second Transmit Beamformer |
|---|---|

*260*

*270*

Signal Processing Unit

Scan Converter

Synchronizing Unit *(140)*

*120*

*220*

| Imaging Transducer | First Transmit Beamformer |
|---|---|

*240*

Receive Beamformer

Display Unit *(180)*

# FIG. 2

**FIG. 3A**

**FIG. 3B**

**FIG. 4**

**FIG. 5A**

**FIG. 5B**

**FIG. 6A**

**FIG. 6B**

**FIG. 6C**

**FIG. 7**

**FIG. 8A**

**FIG. 8B**

**FIG. 8C**

**FIG. 9**

<table>
<tr><td colspan="2"><b>INTERNATIONAL SEARCH REPORT</b></td><td>International application No.<br><b>PCT/KR2013/001671</b></td></tr>
</table>

**A. CLASSIFICATION OF SUBJECT MATTER**

*A61N 7/00(2006.01)i, A61B 8/00(2006.01)i, A61B 8/14(2006.01)i, G01S 15/89(2006.01)i*

According to International Patent Classification (IPC) or to both national classification and IPC

**B. FIELDS SEARCHED**

Minimum documentation searched (classification system followed by classification symbols)
A61N 7/00; A61B 18/00; A61B 8/14; G01B 17/00; A61B 8/00; A61N 7/02; G01S 15/89

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: ultrasonic waves, imaging, transducer, focus, transmission point, synchronization

**C. DOCUMENTS CONSIDERED TO BE RELEVANT**

| Category* | Citation of document, with indication, where appropriate, of the relevant passages | Relevant to claim No. |
|---|---|---|
| A | KR 10-1160958 B1 (SOGANG UNIVERSITY RESEARCH FOUNDATION) 29 June 2012<br>See abstract; claims 1 and 3-5 | 1-18 |
| A | KR 10-2013-0016038 A (YONSEI UNIVERSITY WONJU INDUSTRY-ACADEMIC COOPERATION FOUNDATION) 14 February 2013<br>See abstract; claims 1 and 2 | 1-18 |
| A | JP 07-079972 A (FUJITSU LTD.) 28 March 1995<br>See abstract; claim 1 | 1-18 |
| A | KR 10-2013-0010892 A (MAUI IMAGING, INC.) 29 January 2013<br>See abstract; claim 1 | 1-18 |
| A | KR 10-2012-0010012 A (SOGANG UNIVERSITY RESEARCH FOUNDATION) 02 February 2012<br>See abstract; claim 1 | 1-18 |

☐ Further documents are listed in the continuation of Box C. ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 25 SEPTEMBER 2013 (25.09.2013) | **25 SEPTEMBER 2013 (25.09.2013)** |

| Name and mailing address of the ISA/KR | Authorized officer |
|---|---|
| Korean Intellectual Property Office<br>Government Complex-Daejeon, 189 Seonsa-ro, Daejeon 302-701,<br>Republic of Korea | |
| Facsimile No. 82-42-472-7140 | Telephone No. |

Form PCT/ISA/210 (second sheet) (July 2009)

International application No.

**PCT/KR2013/001671**

| Patent document cited in search report | Publication date | Patent family member | Publication date |
|---|---|---|---|
| KR 10-1160958 B1 | 29/06/2012 | KR 10-2011-0076377 A | 06/07/2011 |
| KR 10-2013-0016038 A | 14/02/2013 | NONE | |
| JP 07-079972 A | 28/03/1995 | JP 3320853 B2 | 03/09/2002 |
| KR 10-2013-0010892 A | 29/01/2013 | JP 2013-520235 A<br>WO 2011-103303 A2<br>WO 2011-103303 A3 | 06/06/2013<br>25/08/2011<br>17/11/2011 |
| KR 10-2012-0010012 A | 02/02/2012 | NONE | |

Form PCT/ISA/210 (patent family annex) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020130021974